# EUROPEAN PATENT SPECIFICATION

(11) **EP 1 949 915 B1**
(45) Date of publication and mention of the grant of the patent: **22.08.2012**
(21) Application number: 08007944.5
(22) Date of filing: 29.04.2005
(51) Int. Cl.: A61M 1/36, A61K 39/395, A61M 1/34, A61P 35/00, A61K 38/19, A61K 38/20

(54) **Method and system to remove soluble TNFR1, TNFR2, and IL2R in patients**
Verfahren und System zur Entfernung von löslichem TNFR1, TNFR2 und IL2R bei Patienten
Procédé et système pour supprimer des TNFR1, TNFR2, et IL2R solubles chez des patients

(30) Priority: 30.04.2004 US 566741 P
(43) Date of publication of application: 30.07.2008
(62) Divisional of application: 05744057.0
(73) Proprietor: BioPheresis Technologies, Inc., Atlanta, GA 30339 (US)
(72) Inventor: Lentz, Rigdon M, 83209 Prien am Chiemsee (DE)
(74) Representative: Lahrtz, Fritz

(56) References cited:
- WO-A-01/37873
- WO-A-99/61085
- DATABASE EMBASE [Online] ELSEVIER SCIENCE PUBLISHERS, AMSTERDAM, NL; 1998, SHIBATA M ET AL: "Changes of cell-mediated immunity with an advance of cancer - Relation to the Th1/Th2 balance and inhibitors of Th1 cytokines" XP008059088 Database accession no. EMB-1998193960 & BIOTHERAPY 1998 JAPAN, vol. 12, no. 5, 1998, pages 715-717, ISSN: 0914-2223
- LENTZ M R: "THE ROLE OF THERAPEUTIC APHERESIS IN THE TREATMENT OF CANCER: A REVIEW" METABOLIC ENGINEERING, ACADEMIC PRESS,, US, vol. 3, no. 1, 2001, pages 40-48, XP001010112 ISSN: 1096-7176
- LENTZ M R: "CONTINUOUS WHOLE BLOOD ULTRAPHERESIS PROCEDURE IN PATIENTS WITH METASTATIC CANCER" JOURNAL OF BIOLOGICAL RESPONSE MODIFIERS, RAVEN PRESS, NEW YORK, NY, US, vol. 8, no. 5, October 1989 (1989-10), pages 511-527, XP001010099 ISSN: 0732-6580
- SELINSKY C ET AL: "Multifaceted inhibition of anti-tumour immune mechanisms by soluble tumour necrosis factor receptor type I" IMMUNOLOGY, BLACKWELL PUBLISHING, OXFORD, GB, vol. 94, no. 1, May 1998 (1998-05), pages 88-93, XP002172135 ISSN: 0019-2805
- Meredith Lentz ET AL: "Reduction of Plasma Levels of Soluble Tumor Necrosis Factor and Interleukin-2 Receptors by Means of a Novel Immunoadsorption Column", Therapeutic Apheresis and Dialysis, vol. 12, no. 6, 1 December 2008 (2008-12-01), pages 491-499, XP55001372, ISSN: 1744-9979, DOI: 10.1111/j.1744-9987.2008.00640.x

## Description

### Background of the Invention

This application claims priority to U.S.S.N. 60/566,741 filed April 30, 2004.

The present invention is generally in the field of enhancing an immune response, and particularly relates to the removal of soluble tumor necrosis factor receptors ("sTNFR1", "sTNFR2") and soluble interleukin 2 receptors ("sIL2") in a patient, such as a cancer patient, to promote inflammation and thereby induce remission of the cancer.

Conventional cancer therapy is based on the use of drugs and/or radiation which kills replicating cells, hopefully faster than the agents kill the patient's normal cells. Surgery is used to reduce tumor bulk, but has little impact once the cancer has metastasized. Radiation is effective only in a localized area.

The treatments can in themselves kill the patient, in the absence of maintenance therapy. For example, for some types of cancer, bone marrow transplants have been used to maintain the patient following treatment with otherwise fatal amounts of chemotherapy. Efficacy has not been proven for treatment of solid tumors, however. "Cocktails" of different chemotherapeutic agents and combinations of very high doses of chemotherapy with restorative agents, for example, granulocyte macrophage colony stimulating factor ("GM-CSF"), erythropoietin, thrombopoetin granulocyte stimulating factor, ("G-CSF"), macrophage colony stimulating factor ("M-CSF") and stem cell factor ("SCF") to restore platelet and white cell levels, have been used to treat aggressive cancers. Even with the supportive or restrictive therapy, side effects are severe.

Other treatments have been tried in an attempt to improve mortality and morbidity. Vaccines to stimulate the patient's immune system have been attempted, but not with great success. Various cytokines, alone or in combination, such as tumor necrosis factor, interferon gamma, and interleukin-2 ("IL-2") have been used to kill cancers, but have not produced significant clinical responses. More recently, anti-angiogenic compounds such as thalidomide have been tried in compassionate use cases and shown to cause tumor remission. In animal studies, compounds inducing a procoagulant state, such as an inhibitor of protein C, have been used to cause tumor remission.

U.S. Patent No. 4,708,713 to Lentz describes an alternative method for treating cancer, involving ultrapheresis to remove compounds based on molecular weight, which promotes an immune attack on the tumors by the patient's own white cells. U.S. Patent No. 6,620,382 to Lentz describes a method of removing molecules of less than 120,000 daltons to provoke an immune response to induce remission. Molecules are removed either using a filter with a molecular weight cutoff of 120,000 daltons or less through which plasma is circulated, or using an immunoglobulin column, containing antibodies to sTNFRs or other cytokine inhibitors. Both ultrapheresis and selective removal of the soluble cytokines have demonstrated reduction in tumor mass in cancer patients.

WO-01/37873 describes ultrapheresis to remove soluble TNF receptor 1 and soluble TNF receptor 2 from a patients plasma to treat cancer.

It is an object of the present invention to provide a method and system for treatment of solid tumors through the selective removal of soluble cytokine receptor molecules producing greater tumor reduction.

### Summary of the Invention

The present invention refers to a sterile device for removal of soluble tumor necrosis factor receptor 1 (sTNFR1), soluble tumor necrosis factor receptor 2 (sTNFR2), and soluble interleukin 2 receptor (sIL2R) from blood or plasma, said device comprising a composition comprising binding partners binding to soluble tumor necrosis factor receptor 1 (sTNFR1), soluble tumor necrosis factor receptor 2 (sTNFR2), and soluble interleukin 2 receptor (sIL2R) immobilized onto a polymeric material in a column or filter.

A method, and system, to induce remission in diseases characterized by excess production of sTNR and interleukin 2R has been developed. The system includes a means for separation of blood into plasma and blood cells, such as a plasmapheresis machine, where the plasma is then treated using a column or filter having immobilized thereon binding partners such as antibodies to sTNFR1, sTNFR2 and sIL2R, or the cytokines or portions thereof which bind to these receptors, until the levels of the soluble cytokine receptors are reduced to below normal, and the treated plasma returned to the patient. This selection of the binding partners represents a significant improvement over earlier systems which included only the sTNFR binding partners, and significantly narrows the options previously discussed with respect to the wide range of other materials that it might be desirable to remove. In the preferred embodiment, the system includes a filter which separates the blood components from the plasma, or filtrate, which is then passaged through a column containing polyclonal antibodies to selected cytokine soluble receptors whch are immobilized in a column containing a material such as SEPHAROSE^{™}. The plasma is circulated through the column until the desired reduction in levels of sTNFR1, sTNFR2, and IL2 is achieved. In the preferred method, patients are treated three to five times a week for four weeks, most preferably daily. The process can be performed alone or in combination with other therapies, including radiation, chemotherapy (local or systemic, for example, treatments using alkylating agents, doxyrubicin, carboplatinum, cisplatinum, and taxol.

In the preferred embodiment, the plasma is treated so that normal levels of circulating soluble cytokine receptors (referred to herein as "inhibitors") are achieved within the first hour of treatment. Treatment is then continued so that levels are reduced below normal and maintained at less than normal levels for a period of at least four to five hours. Clinical studies have demonstrated that it is important to control the flow rate of the plasma through the column. Typical flow rates of plasma through the column are between 10 and 100 ml/min, preferably between 50 and 100 ml/min. This is based on a separation of 100 ml plasma (filtrate)/min from blood passing through the plasmapheresis system at a rate of 300 ml/min to 500 ml/min

### Brief Description of the Drawings

Figure 1 is a perspective view of a column containing immobilized antibodies.
Figure 2 is a schematic of the ultrapheresis process.
Figure 3a is a graph of Pre- and post-treatment sTNFR1 levels during the treatment cycle and amount of receptor eluted from the columns after each procedure (presented in ng/10), n = 132. Figure 3b is a graph of Pre- and post-treatment sTNFR2 levels during the treatment cycle and amount of receptor eluted from the columns after each procedure (presented in ng/10), (n =132). Figure 3c is a graph of Pre- and post-treatment sIL2R levels during the entire treatment cycle and amount of receptor removed from the columns after each procedure (presented in ng/10), (n = 55)
Figure 4 is a graph of s TNFR1, sTNFR2, and sIL2R removal during the procedures with stable device performance (procedures 3 to 12).
Figure 5a is a graph of sTNFR1 and sTNFR2 Concentrations during therapy (n = 8). Figure 5b is a graph of sTNFR1-concentrations in the filtered plasma before and after passing through the immunopheresis column (n = 59).
Figure 5c is a graph of sTNFR2-concentrations in the filtered plasma before and after passing through the immunopheresis column (n = 59).

### Detailed Description of the Invention

### I. Systems

The system for treatment of patients to reduce the level of circulating soluble tumor necrosis factor receptor (sTNFR) 1, sTNFR2, and soluble interleukin 2 receptor (sIL-2) includes:

A device such as a plasmapheresis system for removal of the blood from a patient;

Means for separating the blood into plasma and cellular elements such as the red and white cells, such as a filter or a centrifuge;

Means containing immobilized binding partners for the soluble cytokine receptors, sTNFR1, sTNFR2, and sIL-2, which can be either a column or a filter;

Means for return of the plasma and separated and treated plasma to the patient, which usually consists of a tubing set.

### A. Plasmapheresis Systems

Although it is possible to treat whole blood to remove soluble cytokine receptor inhibitors, it can be preferable to first separate formed elements and plasma and treat the plasma. This provides for fewer potential problems due to damage to the red cells or activation of the white cells as they pass through the column or filter for removal of the inhibitors. Systems for separating blood into the cellular components and plasma are commercially available. A suitable system is the B. Braun Diapact CCRT plasma exchange/plasma profusion controller with plasma profusion tubing. Other extracorporeal blood treatment systems include the Fresenius Hemocare Apheresis system, the Gambo Prisma System and the Asahi and Kurray blood filtration controllers and the Exorim Immuoadsorption Systems.

### 1. Filters

In the preferred embodiment, the plasma is separated by a filter. The filter must be biocompatible, and suitable for contact with blood, without causing excessive activation of platelets or clotting. Devices will typically be either parallel plate filters or capillary membrane filters. These can be adapted from devices currently in use for kidney dialysis. The capillary membrane filters will typically have a surface area of between about 0.25 and 1 m² for use with children and between about 1 and 3 m² for use with adults. The parallel plate filters will typically have a surface area in the range from 0.1 and 2 cm²/ml of blood to be filtered.

The filter membranes will typically be a biocompatible or inert thermoplastic such as polycarbonate, polytetrafluorethylene (Teflon^{R}), polypropylene, ethylene polyvinyl alcohol or polysulfone. It is often desirable to profuse proteins in the lower molecular weight fraction of the plasma, and avoid profusing large macromolecular proteins, such as fibrinogen, alpha 2 macroglobulin, and macroglobulins such as cryoglobulins, over the adsorber. Therefore membrane that possess molecular seiving discrimination in these molecular sizes are desirable. Such membranes ideally have a pore size typically of between 0.02 and 0.05 microns in a capillary membrane filter and of between 0.04 and 0.08 microns in a parallel plate filter. Polysulfone is preferred to ethylene vinyl acetate since it is more gentle towards the blood cells. The actual pore size that yields the desired cutoff is determined based on the fluid flow geometry, shear forces, flow rates, and surface area. The effective cutoff for a capillary membrane filter with a pore size of 0.03 microns is 150,000 daltons, with a sieving coefficient of between 10 and 30%. The filter membrane should be less than about 25 microns, preferably less than about 10 microns, thick. The permeable membrane should not cause blood clotting or otherwise react with the blood.

In a preferred embodiment in which only molecules having a molecular weight of approximately 120,000-150,000 daltons or less are removed from the blood, the filter has a sieving coefficient that removes zero% of the fibrinogen; 10-50% of the IgG; 80-100% of the SGOT and LDH (100,000 mw), and 100% of the sTNFR1 (74,000 as it circulates as a dimer or aggregate).

Suitable devices can be obtained from Asahi Chemical Company, Japan, and Kuraray Co., Ltd, 1-12-39, Umeda, ite-ku, Osaka 530, Japan. The Kuraray 4A or 5A plasma separator is the most preferred plasma separator. Other preferred filters include the Frezenius polysulfone filter and the Kuraray 3A and 2A filters. Staged filters can also be used, which have different pore sizes and/or geometries or surfaces areas, to provide for a "staggered" removal of materials from the blood.

The flow rate of plasma from these systems depends on the blood flow rate and the filter. At a flow rate of 300 ml blood/min (with a range of between 150 and 500 ml/min), the plasmapheresis systems typically yield a plasma flow rate of 100 ml filtrate (plasma)/min. The preferred range of flow rates is between 10 and 100 ml/min, with a more preferred range of between 50 and 100 ml.

### 2. Other Means of Separation

Alternatively, although not at this time preferred, one can use differential centrifugation, to provide for an appropriate separation of blood components.

### 3. Other Blood Treatment System

Alternatively, the matrix of the adsobant column can be constructed in its geomtetry so as to couple the inhibitor binding ligands in microscopic pits on the surface of the bead so as to allow plasma proteins to come in contact with the binding ligand (antibody or peptide) but prevent blood cells from coming in contact with the binding ligand. This system allows for the removal of the desired inhibitors from whole blood and makes the use of a filter unnessary.

### B. Process Controls and Fluid Handling

The patient will typically be connected to the blood processing device using an indwelling venous catheter and and standard intravenous tubing, with connections similar to those used for other extracorporeal blood treatment systems, so that blood can be removed from and returned to the patient. The tubing is connected to a blood pump that controls the flow rate so that in the preferred embodiment one blood volume (based on approximately 7% of the total body weight) is processed over a period of approximately 15-20 minutes. After passing through the blood filter, the plasma filtrate is directed to the inhibitor removal column or filter, then returned from these devices to the patient at either a single catheter site or a second site. Standard microprocessor controls can be used to regulate the blood flow, for example, by monitoring the volume of the blood products being removed, in combination with flow rate monitors and pump speed.

The entire system should first be flushed with saline and then treated with an anticoagulant or anticlotting agent, such as sodium heparin or anticoagulant citrate dextrose ("ACD"), to be sure that there are no locations within the system where blood clotting can occur. Moreover, small amounts of anticoagulants should be continually introduced into the blood stream directed to the blood filter to ensure than no clotting occurs during the filtration process. All of the surfaces of the system which come in contact with the blood and fluids which are infused into the patient must be either sterilized or prepared aseptically prior to commencing treatment.

### C. Columns or Filters for Inhibitor Removal

### 1. Binding Partners

Inhibitors can be removed by binding to either antibodies to the inhibitors or the cytokines which normally bind to the receptors. Selective removal or neutralization of the soluble cytokine receptors (which function as inhibitors of the cytokine) is used to promote a selective, safe inflammatory response against transformed, diseased or autoimmune cells.

The receptors can be removed by binding to the immobilized cytokine, an epitope or fragment thereof which selectively binds to the soluble cytokine receptor, or an antibody to the receptor. As used herein, the term "selectively binds" means that a molecule binds to one type of target molecule, but not substantially to other types of molecules. The term "specifically binds" is used interchangeably herein with "selectively binds" As used herein, the term "binding partner" is intended to include any molecule chosen for its ability to selectively bind to the targeted immune system inhibitor. The binding partner can be one which naturally binds the targeted immune system inhibitor. For example, tumor necrosis factor alpha or beta.can be used as a binding partner for sTNFRI. Alternatively, other binding partners, chosen for their ability to selectively bind to the targeted immune system inhibitor, can be used. These include fragments of the natural binding partner, polyclonal or monoclonal antibody preparations or fragments thereof, or synthetic peptides.

Antibodies can be polyclonal, monoclonal, recombinant, synthetic or humanized. Antibody fragments or single chain antibodies may also be used that bind to the inhibitor to be removed. Polyclonal antibodies are preferred since these have a broader range of reactivity and it is not necessary to have human antibodies since the antibodies are immobilized, not administered to the patient. Typically, the small amount of leaching that is observed does not create a significant risk. The antibodies described in the following clinical study were obtained by immunization of rabbits with sTNFR1, sTNFR2 or sIL2R. The antibodies will typically be reactive with both the soluble and immobilized forms of the receptor, soluble tumor necrosis factor receptor ("sTNF-R") 1 and 2 and soluble interleukin-2 receptor ("sII,-2R").

The antibodies to the receptors can be immobilized in a filter, in a column, or using other standard techniques for binding reactions to remove proteins from the blood or plasma of a patient, or administered directly to the patient in a suitable pharmaceutically acceptable carrier such as saline. As used herein, antibody refers to antibody, or antibody fragments (single chain, recombinant, or humanized), immunoreactive with the receptor molecules.

Antibodies can be obtained from various commercial sources such as Genzyme Pharmaceuticals. These are preferably humanized for direct administration to a human, but may be of animal origin if immobilized in an extracorporeal device. Antibodies may be monoclonal or polyclonal. The antibodies and device should be prepared aseptically so as not to contain endotoxin or other materials not acceptable for administration to a patient.

Antibodies to the receptor proteins can be generated by standard techniques, using human receptor proteins or antigenic fragments thereof. Antibodies are typically generated by immunization of an animal, then isolated from the serum, or used to make hybridomas which express the antibodies in culture. Because the methods for immunizing animals yield antibody which is not of human origin, the antibodies could elicit an adverse effect if administered to humans. Methods for "humanizing" antibodies, or generating less immunogenic fragments of non-human antibodies, are well known. A humanized antibody is one in which only the antigen-recognized sites, or complementarily-determining hypervariable regions (CDRs) are of non-human origin, whereas all framework regions (FR) of variable domains are products of human genes. These "humanized" antibodies present a lesser xenographic rejection stimulus when introduced to a human recipient.

To accomplish humanization of a selected mouse monoclonal antibody, the CDR grafting method described by Daugherty, et al., (1991) Nucl. Acids Res., 19:2471-2476, incorporated herein by reference, may be used. Briefly, the variable region DNA of a selected animal recombinant anti-idiotypic ScFv is sequenced by the method of Clackson, T., et al., (1991) Nature, 352:624-688, incorporated herein by reference. Using this sequence, animal CDRs are distinguished from animal framework regions (FR) based on locations of the CDRs in known sequences of animal variable genes. Kabat, H.A., et al., Sequences of Proteins of Immunological Interest, 4th Ed. (U.S. Dept. Health and Human Services, Bethesda, MD, 1987). Once the animal CDRs and FR are identified, the CDRs are grafted onto human heavy chain variable region framework by the use of synthetic oligonucleotides and polymerase chain reaction (PCR) recombination. Codons for the animal heavy chain CDRs, as well as the available human heavy chain variable region framework, are built in four (each 100 bases long) oligonucleotides. Using PCR, a grated DNA sequence of 400 bases is formed that encodes for the recombinant animal CDR/human heavy chain FR protection.

The immunogenic stimulus presented by the monoclonal antibodies so produced may be further decreased by the use of Pharmacia's (Pharmacia LKB Biotechnology, Sweden) "Recombinant Phage Antibody System" (RPAS), which generated a single-chain Fv fragment (ScFv) which incorporates the complete antigen-binding domain of the antibody. In the RPAS, antibody variable heavy and light chain genes are separately amplified from the hybridoma mRNA and cloned into an expression vector. The heavy and light chain domains are co-expressed on the same polypeptide chain after joining with a short linker DNA which codes for a flexible peptide. This assembly generated a single-chain Fv fragment (ScFv) which incorporates the complete antigen-binding domain of the antibody. Compared to the intact monoclonal antibody, the recombinant ScFv includes a considerably lower number of epitopes, and thereby presents a much weaker immunogenic stimulus when injected into humans.

The cytokine, such as TNF or IL-2, can be immobilized and used to remove the sTNFR and sIL-2. These are the natural binding partners for the receptors. Fragments or "epitopes" (peptide fragments of at least four to seven amino acids in length that can elicit and bind to antibodies to the intact protein) of the cytokines which bind the receptors can also be used. They can be isolated from natural sources or more preferably prepared using standard recombinant technology. Short peptides or fragments can also be prepared using standard synthetic technology. The amino acid sequences and gene sequences encoding the protein are well known.

### 2. Immobilization Substrates

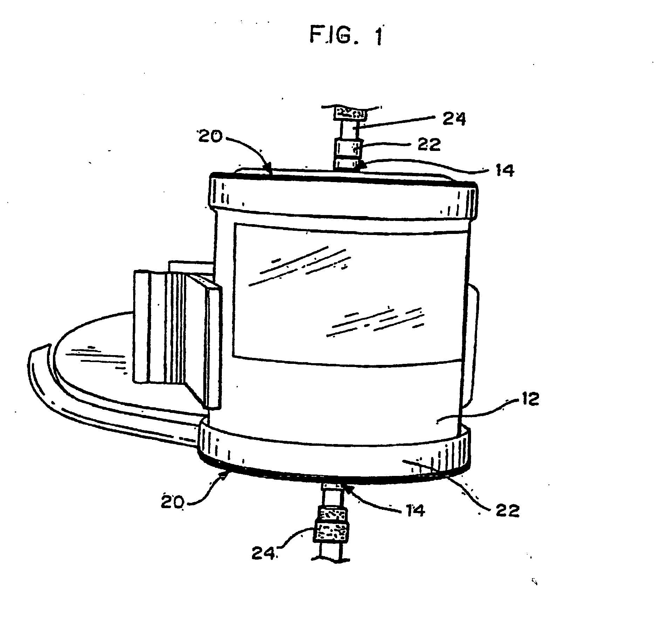

In the preferred embodiment, plasma is circulated through an inert polymeric matrix, such as SEPHAROSE^{™}, sold by Amersham-Biosciences, Upsala, Sweden, within a medical grade polycarbonate housing approximately 325 ml in volume, supplied by Tacoma Plastics, as shown in Figure 1. Other equivalent materials can be used. These should be sterilizable or produced aseptically and be suitable for connection using standard apheresis tubing sets. Typical materials include acrylamide and agarose particles or beads.

Other suitable matrices are available, and can be formed of acrylamide or other inert polymeric material to which antibody can be bound. Standard techniques for coupling of antibodies to the gel material are used.

In another embodiment, the binding partners are immobilized to filter membranes or capillary dialysis tubing, where the plasma passes adjacent to, or through, the membranes to which the binding partners are bound. Suitable filters include those discussed above with respect to separation of blood components. These may be the same filters, having immobilized binding partners bound thereto, or may be arranged in sequence, so that the initial filter separates the blood components and the subsequent filter removes the inhibitors.

In still a third embodiment, the immobilized binding partners are bound to particles that are exposed to the blood or plasma within a mesh or reactor having retaining means. In a particularly preferred embodiment, the particles are highly irregular, so that the binding partners are attached within the invaginations (microscopic pits), either directly using a technique such as cynanogen bromide coupling, or indirectly through a linker such as a polyethylene glycol linker or a binding pair such as avidin and strepavidin, allowing the cells to pass over the particles without risk of reaction with the bound binding partners.

### 3. Columns and Filters

There are three principle embodiments of the device for selective removal of the inhibitors: a column, a filter, or a reactor, all of which contain immobilized binding partners for the inhibitors. The columns or filters are made of a medical grade inert material, preferably a thermoplastic such as a polycarbonate, polyethylene or polypropylene. Filters are the same as those discussed above with respect to separation of the blood components.

The binding partners can be bound to matrix material such as beads for packing of the column or to the filter membranes, on either or both sides of the membranes.

Figure 1 shows the column used in clinical studies to treat cancer patients, as discussed in the following examples. The column 10 includes a housing 12, filters 14 at both the intake 16 and outlet ports 18, and o-ring seals 20 at both ports to seal caps 22 onto the column housing 12. Plugs 24 seal the ports at either end of the column.

The immobilizing binding partner is packed into the column after sterilization or aseptic treatment of the material. Coupling of the antibody to the matrix using a technique such as cyanogen bromide significantly reduces virus due either to removal of the unbound virus during washing or by coupling the virus to the matrix material, which inactivates the bound virus. Due to recent concerns regarding the potential for viruses from the animals used to make polyclonal antibodies, such as the rabbits used to make the antibodies in the following examples, the antibody is bound to the matrix material, the matrix material is placed into a bag which is then spread to provide for maximum exposed surface area and treated by stationary e-beam radiation (24 centi). This can cause up to 25% loss of activity and antibody quantities may have to be increased accordingly. Other known sterilization techniques that may be used, alone or in combination, include washing the matrix material containing immobilized binding partner with glycine at a pH of 2.8 which destroys enveloped virus (two to three log reduction); ultraviolet irradiation which causes a four to five log reduction of all viruses with only about 5% loss of antibody activity. The sterilized or aseptically prepared matrix material is transferred from the bag through a sterile port in the bag directly into the sterilized column port.

Column housings are sterilized prior to packing with immobilized antibody, which is done using aseptic conditions. Columns are filled with 0.1% sodium azide in phosphate buffered saline ("PBS") as a preservative, although other medically equivalent buffers could be used. These are stored refrigerated until use.

Columns may be regenerated by washing with normal sterile saline, elution with 200 mM glycine-HCl pH 2.8, washing with normal sterile saline, then washing with PBS. Other equivalent washing solutions can be used. The column is flushed with multiple volumes of sterile saline prior to use.

### C. Treatment System

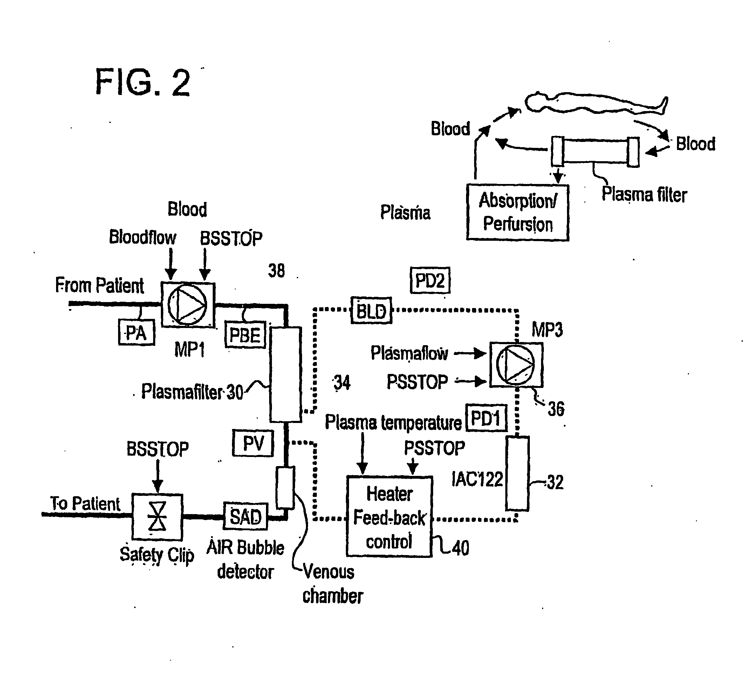

Figure 2 is a schematic of the ultrapheresis system including column. Blood is initially passed through a plasma filter 30; the plasma is passed through the column containing binding partners 32, and then the treated plasma is recombined with the blood cells at 34 for administration back into the patient. Pumps 36 and 38 regulate flow rate through the column 32 and plasma separating filter 30, respectively. A heater 40 maintains temperature control.

### II. Method of Treatment

Patients to be treated are those adults characterized by cancerous tumors, or other diseases characterized by the overproduction and elevated levels of sTNFR1, sTNFR2 and sIL2R, which may include individuals with autoimmune, viral, parasitic, or other disease. Treatment cycles typically consist of three or more treatments per week and/or a total of twelve or more treatments, over a period of time for up to five weeks. Treatment cycles can be repeated as required.

The patient typically requires a dialysis catheter or other device that allows adequate vascular access for treatment. The catheter is connected to the apheresis equipment, which separates the plasma from the formed elements. The plasma is then passed through the filter and returned to the patient. The system and process is depicted in Figure 2. In a preferred embodiment, the plasma is separated through a filter.

The patient is treated for a period of time sufficient to lower the levels of circulating sTNFR1, sTNFR2, and sIL2R. Clinical goals are in the low normal level ranges for these receptors, approximately 750 pg/ml for sTNFR1 and 1250 pg/ml for sTNFR2, and less than approximately 190 pg/mL for sIL-2.

In one embodiment, the levels are reduced to at least 5% less than normal values; in another embodiment, the levels are reduced to at least 10% less than normal values. Circulating levels of the inhibitors frequently rise significantly following treatment, which may be due to shedding by the tumors.

In the preferred embodiment, the plasma is treated so that normal levels of circulating inhibitors are achieved within the first hour of treatment. Treatment is then continued so that levels are reduced below normal and maintained at less than normal levels for a period of at least four to five hours. However, the degree of reduction in the levels of the inhibitors must be balanced by the type of tumor to be treated and the tumor burden.

Lowering the concentration of these receptors induces an inflammatory response against the tumor cells. Evidence of an inflammatory response include fever, tumor specific inflammatory pain, tumor swelling and tumor necrosis. Other problems that can occur include tumor lysis syndrome, which can be treated with standard medical management by qualified physicians.

Patients can be treated with combination therapy. In one preferred embodiment, the selective removal of inhibitors is combined with an immunostimulant, such as a vaccine against tumor antigens, a cytokine to stimulate the immune system or activate dendritic cells, or compounds that block factors such as fibroblast derived growth factor (FDGF), TGF beta, or EGRF. Immune system activation can also be achieved by selective removal of IL-4 and/or IL-10 to drive the cellular mechanism. Other treatments include administration of hyperthermia, radiation or chemotherapeutic agents, although the latter two are typically not preferred since these can reduce the ability of the immune system to kill the tumors.

The present invention will be further understood by reference to the following clinical study report.

### Example 1: Clinical Study of the treatment of cancer patients with plasmapheresis using Anti-TNFR1, Anti-ThTFR2, and Anti-IL-2R Immobilized Antibodies in a Column.

Secretion of TNFα and interleukine-2 that bind via specific receptors to the tumor cell and induce cell death by aptoptosis is the normal response of the immune system in its constant fight against cancer growth. However, local secretion of high levels of soluble receptors for tumor necrosis factor alpha (sTNFR1 and sTNFR2) and interleukin-2 (sIL2R) are believed to be an effective mechanism by the tumor cell to locally block the attack and destruction by the immune system. Systemic removal of these inhibitors by means of extracorporeal apheresis with the goal to reduce the local inhibitor concentrations below the tumor-protective threshold has, therefore, been considered to be a potential therapeutic measure for cancer treatment.

While this approach had primarily been followed in the past with unspecific removal of proteins as defined by molecular weight ranges by means of a method called "Ultrapheresis", a more specific approach is the use of immobilized antibodies to sTNFR1, sTNFR2, and sIL2R in the apheresis procedure. An apheresis adsorption column containing specific antibodies bound to a polymer matrix has been developed for this approach. Such a device will have to demonstrate its efficacy in receptor binding and should also present with an acceptable risk-benefit profile.

The purpose of this study was to assess the efficacy and safety of the new Biopheresis column to reduce circulating sTNFR1, sTNFR2, and sIL2R in the plasma of cancer patients during the apheresis procedure.

### The Column IAC122

The Immunopheresis column IAC122 is a sterile immune adsorbent product designed to remove soluble inhibitors to pro-inflammatory cytokines from the blood. It is designed to be used in conjunction with commercially available approved extracorporeal blood treatment systems. (e.g. Diapact CRRT device, B. Braun, Fresenius Hemocare Apheresis, Exorim Immuoadsorption Systems.). The device is intended only to be sold on the order of and used only by physicians with experience in the use of Immunoadsorption techniques. The Immune adsorption column is intended to remove soluble pro-inflammatory cytokines which are known to be overproduced in certain disease states like cancers, where they are a major cause of immune tolerance of tumor associated neo-antigen. In clinical application in cancer patients the removal of these inhibitors/shed receptors may produce tumor specific inflammation which can lead to tumor destruction.

The column housing is a 325 ml volume medical grade polycarbonate device (PNS-400146-Fresenius HemoCare, INC). The column matrix is composed of Sephrose 4B beads and polyclonal rabbit antibodies against pro-inflammatory cytokine inhibitors (soluble receptors to tumor necrosis factor alpha (TNF) and interleukine 2 (IL2)). Therefore, the essential components for manufacturing are Sepharose, purchased as sterile product from Amersham-Biosciences (Upsala, Sweden), antibodies to TNF receptors and IL2 receptor that are sterilized by filtration (Eurogentec, Liege, Belgium), and a polycarbonate housing (Fresenius, St. Walin), sterilized by autoclave. Sterile components and aseptic technique during the production, as well as final product testing of each column or column production lot are central to the safety of this medicinal device product. Each column is constructed under aseptic conditions according to the GMP. Each column is individually tested for sterility and endotoxin level post manufacture. Each column is filled with 0.1% Sodium Azide (NaAzide) in PBS and maintained between 4-8° C prior to clinical use. A picture of the device is shown in Figure 1.

The intended purpose of the device is to serve as an adsorption column in clinical apheresis procedures. The column is part of an extracorporeal circuit using a standard plasma perfusion machine that removes blood from patients, separates the plasma by filtration, passes the filtered plasma through an adsorption column and then return the combined plasma and cell fractions to the patient in a continuous loop system (see also Figure 2). The adsorptive material in the column is constructed to specifically bind two kinds of soluble receptors to Tumor Necrosis Factor α (sTNFR1 and sTNFR2) and also to bind soluble receptors to interleukine 2 (sIL2R). The goal of using these columns in apheresis procedures is to remove those inhibitors from the blood that are known to protect tumor cells against destruction by the host immune system.

Indications for use of the device are disease conditions where patients may have a clinical benefit from removal of sTNFR1, sTNFR2, and sIL2R (e.g. metastatic cancer).

If used in accordance with the Instructions for Use, there are no contraindications for the use of this device.

The device has been shown in clinical and laboratory studies to effectively remove sTNFR1, sTNFR2 and sIL2R from the filtered plasma. Lowering the concentration of these receptors during an apheresis procedure should result in the induction of an inflammatory response against the tumor cells. Therefore, signs and symptoms of tumor inflammation have been reported from the clinical study (e.g. fever, tumor specific inflammatory pain, tumor swelling, and tumor necrosis, see also 5.5. Safety Analysis). Prolonged use of the device or treatment of large tumors (> 500 g) may in case of successful induction of inflammatory response lead to an excessive overload of proteins resulting from tumor destruction, which may result in a tumor lysis syndrome with the risk of kidney insufficiency, acute tubular necrosis, acute respiratory deficiency syndrome, disseminated intravascular clotting, and death.

### Study Objectives

The primary objective of this study was to lower plasma levels of sTNF-R1 and sTNF-R2 to the lower end of the normal range (750 pg / ml for R1 and 1250 pg /ml for R2 receptors in citrate plasma) during the procedure. The amount of plasma processed to achieve this level of reduction must have been empirically derived for each patient but was estimated to be an amount of plasma roughly equivalent to one extracellular water volume. This was calculated using body mass (approximately 20% of body mass in kilograms expressed in liters).

The secondary objective was to describe all clinical effects resulting from immunoadsorption (LA) in patients with metastatic cancer using the B. Braun Diapact plasma profusion system with the immunoaffinity column inserted into the plasma circuit. Another secondary objective was to specifically collect subjective and objective evidence of tumor inflammation and tumor necrosis and/or resolution as measured by CAT scan, NMR, and or bone scans or Xrays of osseus metastatic lesions of visceral tumors, or direct measurement of surface tumors.

The serum level of soluble sIL2-receptor was recorded to document possible changes of these levels after apheresis treatments.

Another secondary objective was to assess the safety of the device use by documenting all adverse events associated to the treatment procedures.

### Methodology

This was a single site, open non randomized study to observe and document the lowering of plasma concentrations of sTNFR-1 and sTNFR-2 and the possible effect on tumor mass in patients with metastatic cancer. The study consisted of a total of twelve treatments that took place within five weeks. About 1-10 days after the Baseline visit (V1), a catheter placement Visit (V2) was conducted. This visit was followed by 12 treatment visits (V3-V14), which took place within four weeks, three visits per week. The Final visit (V 15) was performed one day after the last treatment visit.

After the final visit all laboratory data and tumor assessment data was evaluated and a medical recommendation was given to the patient by the investigator. In case the apherese-procedure showed positive changes against the tumor, the patient was given the opportunity to repeat the treatment up to three cycles. A time period of one to three months was allowed between two treatment procedures.

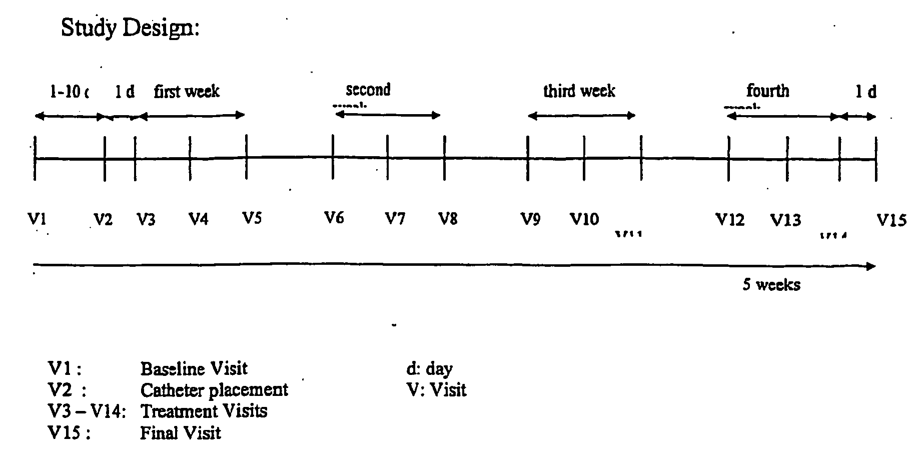

### Eligibility and Enrollment

To be considered eligible to participate in the study, a patient had to meet the following inclusion and exclusion criteria:

### Inclusion criteria

1. Patients with biopsy proven metastatic or recurrent cancer that has failed to respond to standard systemic chemotherapy and / or hormonal therapy.
2. Patients with tumor that is uni-dimensionally measurable.
3. Patients with Kamofsky Status of 70% or greater. (See Appendix A)
4. Patients with life expectancy greater than 3 months.
5. Patients age > 18 years.
6. Patients with measurable plasma s1NF -R1 concentrations and/orsTNFR-2 concentrations that are measurable.
7. Patients with the ability to understand the investigational nature of the trial and sign informed consent prior to any study procedures.
8. Patients who have had progression or recurrence of disease after having received conventional therapy, or have refused these therapies under informed consent.
9. Patients must have an attendant (friend or family member) accompanying them during each treatment visit on all four-week treatment cycles.

Written informed consent must be obtained for all patients.

### Exclusion criteria

1. Patients with an approximative cumulative tumor mass greater than 1000 gms estimated by CAT scan or physical examination at the discretion of the investigator, to decrease the risk of tumor lysis syndrome..
2. Patients with inadequate renal and hepatic function as evidenced by serum creatinine greater than normal value or serum bilirubin greater than normal value.
3. Patients with a positive result, when screened for infection with Hepatitis A, Hepatitis B, Hepatitis C, and HIV
4. Patients with prior radiation, chemotherapy, hormonal or immune therapy within 30 days of protocol entry
5. Females who are pregnant or lactating. In addition, females of childbearing potential who do not agree to use an appropriate method of birth control. Appropriate methods of birth control include, abstinence, oral contraceptives, implantable hormonal contraceptives (Norplant), or double barrier method (e.g., diaphragm plus condom).
6. Patients with a history of or radiographically proven active tumors growing in C.N.S. despite radiation therapy will be excluded.
7. Patients with congestive heart failure will be excluded.
8. Patients with active infectious diseases requiring antibiotics within 30 days of protocol entry will be excluded.
9. Patients with co-existing second malignancies will be excluded.
10. Patients who have not received prior conventional treatment or who have not refused same under informed consent
11. Patients with clinically significant hypotension, resting systolic blood pressure <100 mmHg will be excluded.
12. Patient with a history of myocardial infarction in the last 6 months or with uncontrollable angina.
13. Patients with known hypersensitivity or allergy to rabbits, latex and x-ray contrast medium.
14. Patients with hematocrit less than 30%, WRC less than 2500/pl, and platelet counts less than 100,000/pI will be excluded.
15. Patients with measurable tumor only in sites of previous radiation therapy will be excluded.
16. Patients with tumor associated with hollow viscera will be excluded.
17. Patients with known hypersensitivity to heparin.
18. Patients who participated in the trial three times
19. Patient with a history of severe or multiple allergies.

12 patients were recruited. Three of these patients completed a second treatment cycle. Therefore, 15 treatment cycles can be evaluated for efficacy. The patients characteristics are given in the result section.

### Data Acquisition

The study was conducted in compliance with the declaration of Helsinki, all legal and ethical regulations, and in accordance to the guidelines of Good Clinical Practice. Patient's histories were provided by the patients themselves or from their treating oncologist. Original data and original laboratory results were entered into the Case Report Forms by the investigator. The data used in this interim analysis was collected after appropriate monitoring by IKFE CRO and query clarification. The data was entered into a database and analysed descriptively as given below.

### Statistical methods

The data provided by the IKFE CRO was analysed by means of methods of descriptive statistics, i.e. the data is presented in appropriate tables and graphs. One intention of the study was to demonstrate efficacy of the IAC columns in reducing sTNFR1, sTNFR2 and sILR concentrations in plasma during immunopheresis procedures. Therefore, mean values of normally distributed receptor values before and immediately after the study procedure were calculated and compared by means of student's T-Test. A p-value < 0.05 was considered statistically significant. In some cases, additional samples were taken to allow for establishment of the receptor lowering curves during the procedures. Nominal data (e.g. patient's s _ characteristics and adverse events) were listed and are presented in tables.

### Screening visit (V1)

Prior to any study procedure a signed informed consent was obtained from each patient and a unique patient number was assigned. To assess the eligibility of the patient, he/she was evaluated for inclusion/exclusion criteria; a complete medical history was taken and physical and laboratory examinations were conducted (see below "Clinical Variables" and "Laboratory Parameters"). The demographic data, the nature of any conditions present at the time of the physical examination, any preexisting conditions, concomitant medications and treatments were documented.

A tumor assessment was conducted by CAT Scan, NMR or direct measurement of surface tumors. Patients were asked to provide such data if possible from their treating physician. Laboratory parameters or ECGs that had been determined within 10 days prior to the screening visit were acceptable at the discretion of the investigator to avoid unnecessary blood draws.

The patients received a patient identity card and the 24-hour rescue telephone number at the end of the visit.

### Catheter placement visit (V2)

Patients meeting eligibility requirements had vascular access via the subclavian vein. Catheters used were standard dialysis vascular access catheters HEMOACESS (15 - 20 cm, Hospal, Lyon, France)), or equivalent. When hemostasis from catheter placement was assured and when the right position of the catheter by chest X-ray was verified, ideally 24 hours after placement, the initial immunopheresis procedure was performed.

### Treatment visits (V3-V14)*

An immunopheresis treatment was usually performed 3 times a week. Up to one calculated extra cellular water volume (where ECWV = 20% total body weight in kilograms expressed in liters) was treated daily in an effort to reduce the sTNF-R1 to a low normal (750 pg/ml in citrate plasma), and a sTNF-R2 to a normal range (1250 pg/ml in citrate plasma). Pre and post treatment 1NF-R1 and TNF-RZ levels were obtained. Additional measurements of these two parameters were upon discretion of Investigator. The goal was to reduce these levels during the procedures for 3 of 7 hours in all four weeks. The serum levels of sIL2-receptor were obtained pre and post treatment every second visit (V3, V5, V7, V9, V 11, V14). Once a week, a blood chemistry panel was measured and a tumor assessment was performed by clinical investigation. All information about Adverse events were documented and the study diary was reviewed on each treatment day.

A scheme of the treatment setting of an immunopheresis procedure using the Diapact CCRT-Machine (BBraun Medizintechnik, Melsungen, Germany) and the Evaflux plasma filter (MPS Medical Product Services GmbH, Braunfels, Germany) is given in Figure 2.

The following was monitored during patient's treatments. Additional measurements could be performed at the discretion of the investigator.
a) Heart rate approximately every 60 minutes
b) Clotting time approximately every 60 minutes
c) Temperature approximately every 60 minutes
d) Respiratory rate pre and post treatment
e) Blood pressure approximately every 60 minutes
f) Pressures, flow rates, filtrate and replacement solution volume, and alarm conditions (if any) within the extra corporal circuit, approximately every 30 minutes.
g) Blood samples for the inhibitor concentration will be drawn at the start of treatment, and at the end of treatment

Throughout the procedure, it was sometimes necessary to regenerate the column. The columns were regenerated during an immunopheresis procedure after 9 liters of plasma had run over the column. The 325 mL columns were washed with 1 liter 1 sterile saline, eluted with 1 liter 200 mM glycine-HCI (pH 2.8), and then rewashed with 1 liter sterile saline before restarting the apheresis procedure. All columns were regenerated as the last step in the daily apheresis procedure. This final regeneration step required an initial wash with 2 liters normal sterile saline, elution with 1 liter 200 mM glycine-HGI (pH 2.8), a rewashing with 2 liters normal sterile saline, and finally a washing with 1 liter PBS plus 0.01% sodium azide. The column was then stored at 2 to 8 C in PBS plus 0.01% sodium azide solution until next use. Prior to clinical application, the column containing PBS plus 0.01 % sodium azide was flushed with 9 column volumes of normal sterile saline.

Patients were monitored for 1 - 3 hours (average of 2 hours) after termination of the apheresis procedure in the clinical unit. Vital signs were recorded and the patient seen by the physician prior to discharge. Before the patient was discharged after the first treatment day, he/she and his/her family member were instructed, whom they could contact in case of an emergency.

Afterwards the patient was discharged to the home setting with their attendant. The family member noted temperature, pain, and general health until the next treatment visit. This information was recorded in a patient diary and reported to and recorded by the physician at the next study visit. Patients reported all physical complaints to the on-call clinical personnel. Attending physician was responsible for all aspects of patients medical welfare post procedure and made every effort to document all effects of the procedure whether adverse or not in his daily progress notes. Nurses, patients or their family attendants at night recorded patient temperature every 4 hours into the patient's dairy.

At the final visit the patient underwent the following examinations, similar to the Baseline visit:
- physical examination
- laboratory assessment
- Vital parameters
- Removal of the catheter

The patient received a letter for his primary physician, where the order was given that a tumor assessment (CAT scan, or NMR or tumor assessment by physical exam, sTNF-R1 and R2 levels) had to be conducted in the following four weeks. The patient and the primary physician were asked to inform the investigator about the findings of this tumor assessment as soon as possible.

The investigator had to analyze all available data (laboratory data, tumor assessment data) in the following weeks. The determination of tumor activity was made by measuring disease objectively, using CAT scans, NMR's, and direct measurement of surface tumors. In case the apherese procedure showed any sign of positive effects on tumor cell mass, the patient was contacted and asked, if he/she would like to start with a second (third) study treatment.

If the patient had completed the study procedure, a follow up will be started:
a) Patients whose tumors failed to respond returned to their attending physicians for consideration of other therapies. The treating physician or his designate contacted the patient's physician monthly and made a note in the patient's research chart to determine time to progression and date of death.
b) Patients who successfully completed the treatment protocol with an evaluation of at least a minimal response were evaluated for at least 5 months to assess the durability (defined as time to disease progression and overall survival) of the treatment

### Laboratory measurements

Efficacy variables (sTNFR-1,sTNFR-2 and sIL-2) were determined from the patients serum before and after the treatment procedure at the central laboratory (ikfe Lab) by means of the following GLP-validated methods:
Human sTNFR1 and sTNFR2 Immunoassays (R&D Systems Inc., 614 McKinley Place NE, Minneapolis, MN 55413,USA), for the quantitative determination of human soluble tumor necrosis factor receptor 1 & 2 (sTNFR1 and sTNFR2) concentrations in cell culture supernate, serum, plasma, and urine.
Human sIL-2 receptor ELISA (R&D Systems Inc., 614 McKinley Place NE, Minneapolis, MN 55413,USA). The assay is a solid phase enzyme amplified sensitivity immunoassay (EASIA) for the determination of soluble IL-2 receptors levels in human serum, plasma or cell culture supernate.

### Results

The study was conducted in strict compliance with the Declaration of Helsinki, local and national legal and ethical regulations, and in accordance with the guidelines of Good Clinical Practice.

Altogether, the data from 12 patients and 15 planned treatment cycles (planned 180 treatment procedures) were included into this analysis. Efficacy analysis was performed with the data from all completed treatment procedures (150 procedures). Analysis of changes of sTNFRs overtime relate to the amount of consecutive treatment procedures and was possible with the data of 11 treatment cycles completed per protocol (132 treatment procedures). All data from all patients were included into the safety analysis and for the risk benefit assessment.

The demographic data of all patients is listed in table 1. All patients except one suffered from severe end stage metastatic cancer and were pretreated with either chemotherapy, surgery, radiation therapy, or combinations thereof in their history.

**Table 1.: Patients Characteristics**

| Pat. No. | Initials | Gender | Age | Type of Cancer | Remarks |
|---|---|---|---|---|---|
| 1 | RK | F | 55 | Non-small Cell Lung cancer | Drop out (5 procedures) |
| 2 | ST | F | 41 | Breast Cancer | 2 Cycles |
| 3 | CB | M | 47 | Renal Cell Carcinoma | 2 Cycles |
| 4 | RN | M | 54 | Pancreas Carcinoma | Drop out (4 procedures) |
| 5 | DP | M | 59 | Squamous Cell Carcinoma | 2 Cycles |
| 6 | RB | F | 53 | Breast Cancer | 1 Cycle |
| 7 | KK | M | 34 | Malignant Melanoma | Drop out (9 procedures) |
| 8 | AB | F | 34 | Breast Cancer | 1 Cycle |
| 9 | NO | F | 59 | Breast Cancer | 1 Cycle |
| 10 | JD | F | 56 | Breast Cancer | 1 Cycle |
| 11 | GA | F | 46 | Breast Cancer | Drop out (no procedure) |
| 12 | MD | F | 49 | Breast Cancer | 1Cycle |
| Total | | 8 F/4 M | 48.8±8.8 | | |

| | | | | | |
|---|---|---|---|---|---|
| Underlying disease was breast cancer in 7 cases, one lung cancer, one renal cancer, one pancreas cancer, one squamous cell carcinoma, and one malignant melanoma. Narrative summaries of the patients histories are given in section 5.5. Reason for early termination were death (patient 1), patient decision (patient 4), and detection of an exclusion criteria (patients 7 and 11). Detailed information about the serious adverse event in this study are given in section 5.4. | | | | | |

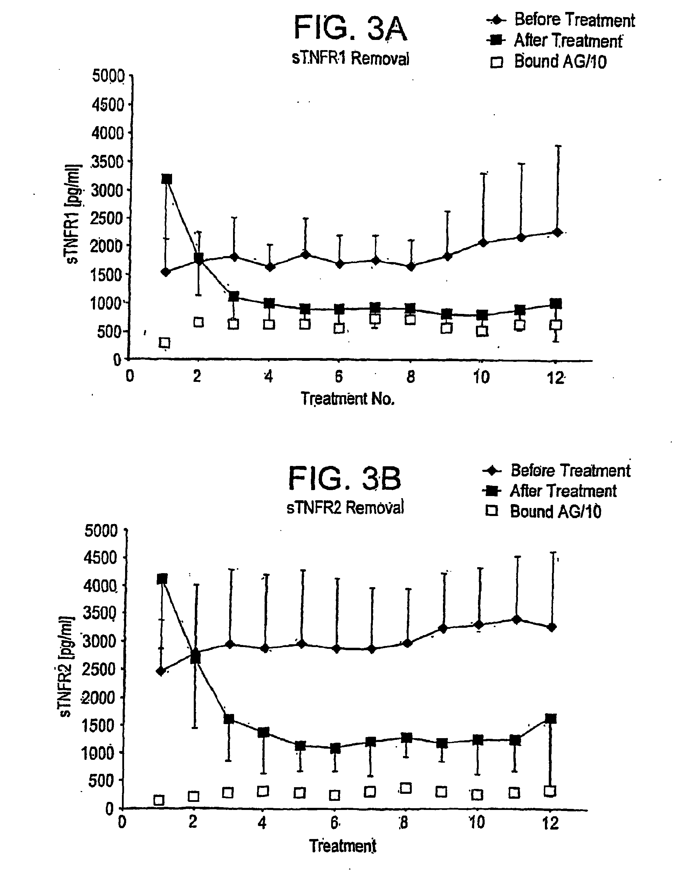

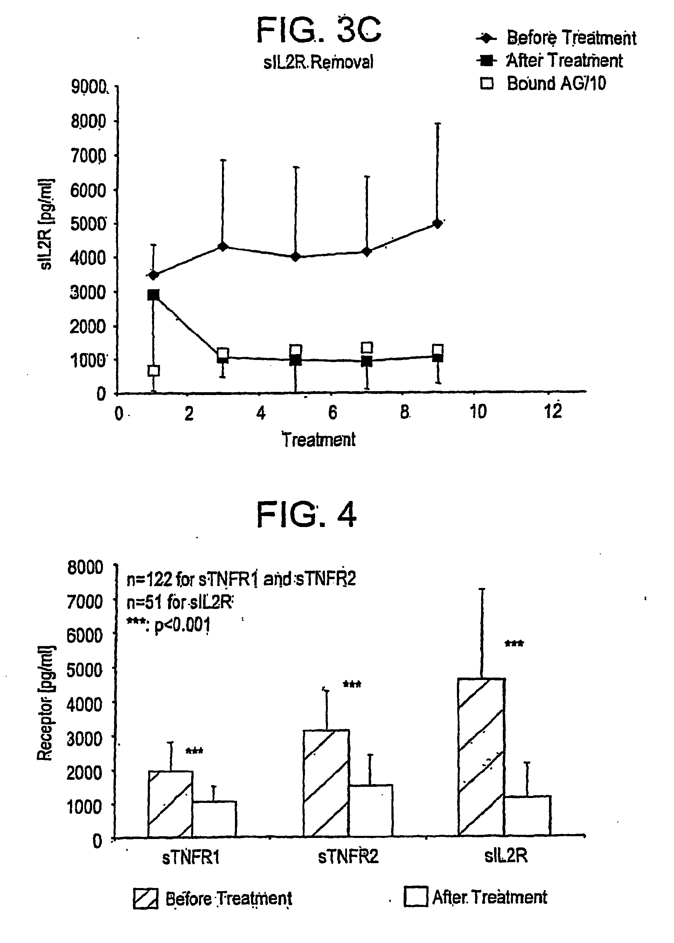

A total of 150 procedures could be included into the efficacy analysis. The plasma concentrations of all three receptors before and after all procedures is given in Table 2. A graphic presentation of the receptor concentrations by treatment number is given in Figures 3a-3c. There was a clear relation between the number of consecutively applied therapeutic procedures and the effect in lowering the sTNFR1, sTNFR2, and sIL2R plasma concentrations. While clinical response of the tumor to the aggressive attempt to lower the soluble receptors lead to a significant increase in the post-treatment receptor levels at the end of the first treatment day, no such effect could be seen at the second treatment day. After the third treatment day plasma concentrations were consistently lowered for all receptors until the end of the treatment cycle. The mean receptor concentrations before and after treatment for the period of stable performance (treatment 3 to treatment 12) is given in Table 3. Not surprisingly, the tumor cells recovered between the treatments and pre-treatment levels were usually at the same levels throughout the procedures. In total, 128 completed treatment procedures could be evaluated for this analysis.

**Table 2 Receptors concentrations at all treatment procedures (n=150)**

| Receptor | Before treatment | After treatment | p-value |
|---|---|---|---|
| sTNFR1[pg/ml] | 1919±828 | 1346±1033 | p<0.001 |
| sTNFR2 [pg/mI] | 3074±1151 | 1870±1291 | p<0.001 |
| sIL2R[pg/ml] | 4745±1692 | 2611±1766 | p<0.001 |

**Table 3 Receptors concentrations at treatment procedures 3 to 12 (n=128)**

| Receptor | Before treatment | After treatment | p-value | % |
|---|---|---|---|---|
| sTNFR1 [pg/ml] | 1948±853 | 1023±466 | p<0.001 | -47.5 |
| sTNFR2 [pg/ml] | 3153±1153 | 1479±894 | p<0.001 | -53.1 |
| sIL2R [pg/ml] | 4845±2711 | 1219±998 | p<0.001 | -74.8 |

The amount of receptors removed from the plasma of the patient was dependent on the volume of filtered plasma. For medical reasons (development of inflammatory response with corresponding symptoms), the initial treatment was carried out carefully and with low plasma flow rates and volumes. In the later treatment phase, plasma volumes of up to 18 1 could be achieved. After each 9 1 of filtered plasma, a regeneration of the columns was performed. The bound material was eluted from the columns by glycine-HCl buffer (ph 2.8). The combined fraction of these cleaning solutions for each patient and procedure were further analyzed by immunoassay to calculate the overall amount of removed receptors. The amount of receptors removed from the columns by regeneration after each treatment procedure is given in Table 4 and in Figures 3a-3c.

**Table 4: Calculation of removed receptor amounts by treatment cycle and achieved plasma filtration volume**

| Procedure No | Plasma Volume [1] | Removed sTNFR1 [ng] | Removed sTNFR2 [ng] | Removed sIL2R [ng] |
|---|---|---|---|---|
| 1 | 4.12±5.31 | 2950 | 1335 | 6997 |
| 2 | 4.95±3.20 | 6755 | 2044 | - |
| 3 | 9.59±5.97 | 6453 | 2811 | 12058 |
| 4 | 11.68±5.35 | 6403 | 3099 | - |
| 5 | 12.56±5.19 | 6436 | 2786 | 10927 |
| 6 | 13.11±4.88 | 5714 | 2400 | - |
| 7 | 14.56±3.61 | 7439 | 3009 | 10499 |
| 8 | 15.31±3.19 | 7314 | 3683 | - |
| 9 | 15.47±3.24 | 5894 | 2810 | 10368 |
| 10 | 15.64±3.12 | 5474 | 2273 | - |
| 11 | 15.11±4.54 | 6807 | 2368 | - |
| 12 | 14.56±4.45 | 6815 | 2864 | - |
| | | | | |
| Mean±STD | 12.22±4.02 | 6204±1184 | 2623±594 | 10170±1984 |
| | | | | |

The analysis showed that the capacity of the columns to remove the three inhibitors to TNF-α and IL-2 remained stable and did not decline during the treatment cycles. Due to the procedure number dependency of the receptor lowering effect, an additional analysis was performed to assess the efficacy of the columns during the periods of stable receptor removal (i.e. during the procedures 3 to 12). It is assumed that the immune system achieved a kind of stabilization in its fight against the tumor cells. This analysis could be performed with all suitable procedure data (n = 122 for sTNFR1 and sTNFR2, and n = 51 for sIL2R).

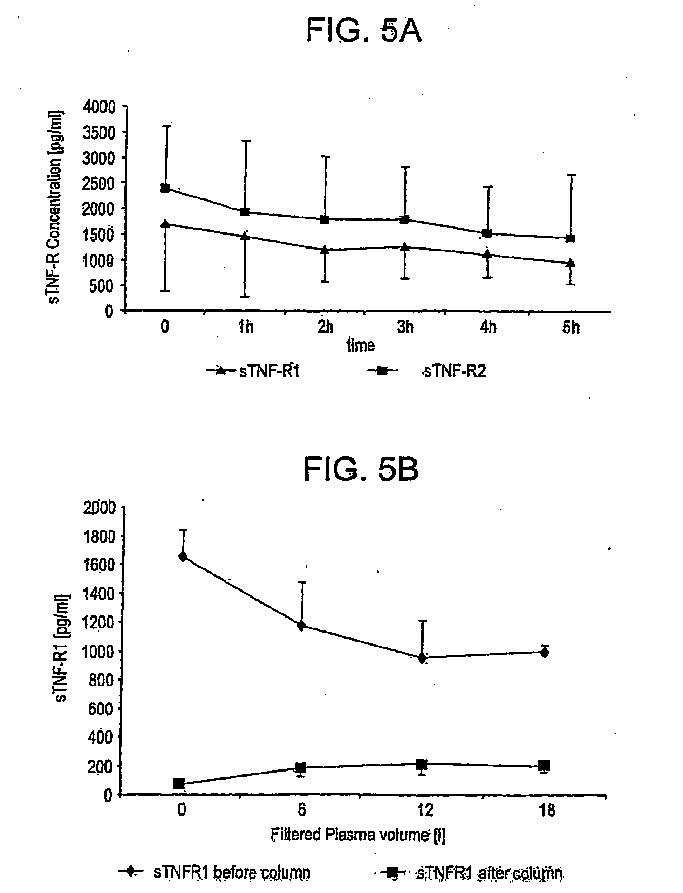

During the period of stabilization of the immune system (procedures 3 to 12), the columns were able to lower the amount of receptors significantly by 44 % for sTNFR1, 52 % for sTNFR2, and 77 % for sIL2R. See Figure 4. In addition to the samples collected as given in the protocol, additional samples were taken during eight procedures to assess the kinetics of sTNFR1 and sTNFR2 decrease in patient plasma during the initial time course of the treatment procedures. For both receptors a steady decrease could be shown in correlation to the filtered plasma volume. The results are given in Figure 5a.

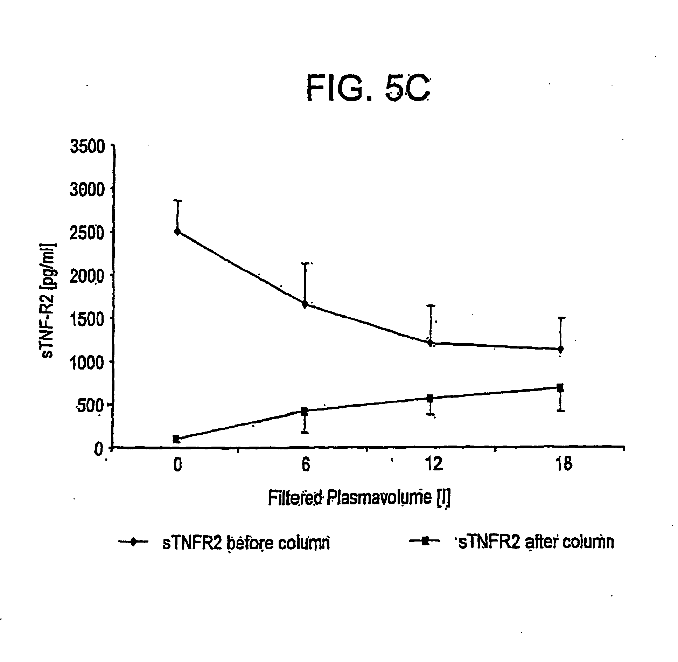

Samples were also taken from the filtered plasma at the machine before the plasma entered the adsorption column and immediately thereafter. The results of these investigations are given in Figures 5b and 5c. It could be shown that both sTNFRs are bound effectively during the immunopheresis procedure, which led to a constant decrease over time in the plasma of the patients. Even after large amounts of filtered plasma passed through the adsorption column, it still maintained significant efficacy in receptor binding. However, it was concluded from these results that a column regeneration is recommended after 9 of filtered plasma to increase the efficacy of the procedure.

As can be expected by the nature of the underlying severe diseases, numerous adverse events were documented during the study. The adverse events can be partly related to the therapy and the majority of these events are indicators of the clinical efficacy of the study treatment procedure to induce an immune response (e,g, fever, shaking etc.). The majority of the overall adverse events, however, is related to the underlying cancers requiring pain medication and other drugs to ease the patients burden. In total, 244 adverse events (AE) and 3 serious adverse events (SAE) including two cases of death were observed. In total, four drop outs were seen in the study (patients 1, 4, 7, 11). Reasons for drop out were death (patient 1), patients decision followed by rapid death (patient 4), development of central nervous metastasis (patient 7), and non-compliance with the inclusion criteria (creatinine > 1.1 mg/l, patient 11).

All receptor concentrations (sTNFR1, sTNFR2, and sIL2R) were significantly reduced during the apheresis procedure by involving the new immunpheresis column IAC122 (by 44 %, 52 %, and 77 %, respectively). The columns proved to be reliable in their performance during the entire treatment cycles. Frequent regeneration of the columns during and after the treatment procedures did not result in a loss of receptor removal capacity. No column had to be removed for reasons of inefficacy or other technical issues. In all patients, receptor reduction lead to clinical symptoms of inflammation and some indications have been collected that this can lead to an improvement to the underlying disease.

## Claims

1. A sterile device for removal of soluble tumor necrosis factor receptor 1 (sTNFR1), soluble tumor necrosis factor receptor 2 (sTNFR2), and soluble interleukin 2 receptor (sIL2R) from blood or plasma, said device comprising a composition comprising binding partners binding to soluble tumor necrosis factor receptor 1 (sTNFR1), soluble tumor necrosis factor receptor 2 (sTNFR2), and soluble interleukin 2 receptor (sIL2R) immobilized onto a polymeric material in a column or filter.

2. The device of claim 1, wherein the binding partner is selected from the group consisting of
a.) polyclonal or monoclonal antibody binding to sTNFR1, sTNFR2, or sIL2R;
b.) antibody fragment (single chain, recombinant, or humanized antibody) binding to sTNFR1, sTNFR2, or sIL2R;
c.) cytokine binding to sTNFR1, sTNFR2, or sIL2R;
d.) fragment of cytokine binding to sTNFR1, sTNFR2, or sIL2R; and
e.) synthetic peptide binding to sTNFR1, sTNFR2, or sIL2R.

3. The device of any one of claims 1 or 2, wherein the polymeric material is selected from the group consisting of
a.) a column;
b.) beads for packing of a column;
c.) a filter;
d.) a filter membrane, on either or both sides of the membrane;
e.) a capillary dialysis tubing;
f.) a matrix; and
g.) a reactor.

4. The device of claim 1, further comprising means for separating the blood into plasma or ultrafiltrate.

5. The device of claim 1, wherein the polymeric material having binding partners immobilized thereon has been treated to reduce viable or infectious virus.

6. The device of claim 5, wherein the material has been treated by e-beam sterilization to inactivate or cross-link virus particles or components thereof.

## Patentansprüche

1. Sterile Vorrichtung zur Entfernung von löslichem Tumornekrosefaktor-Rezeptor 1 (sTNFR1), löslichem Tumomekrosefaktor-Rezeptor 2 (sTNFR2), und löslichem Interleukin 2-Rezeptor (sIL2R) aus Blut oder Plasma, wobei die Vorrichtung eine Zusammensetzung, welche Bindungspartner, die an löslichen Tumomekrosefaktor-Rezeptor 1 (sTNFR1), löslichen Tumornekrosefaktor-Rezeptor 2 (sTNFR2), und löslichen Interleukin 2 Rezeptor (sIL2R) binden, immobilisiert auf einem polymeren Material in einer Säule oder Filter umfasst.

2. Vorrichtung nach Anspruch 1, wobei der Bindungspartner ausgewählt ist aus der Gruppe bestehend aus
a) polyklonaler oder monoklonaler Antikörper bindend an sTNFR1, sTNFR2, oder sIL2R;
b) Antikörperfragment (Einzelkette, rekombinanter oder humanisierter Antikörper) bindend an sTNFR1, sTNFR2, oder sIL2R;
c) Cytokin bindend an sTNFR1, sTNFR2, oder sIL2R;
d) Cytokin-Fragment bindend an sTNFR1, sTNFR2, oder sIL2R; und
e) synthetisches Peptid bindend an sTNFR1, sTNFR2, oder sIL2R.

3. Vorrichtung nach einem beliebigen der Ansprüche 1 oder 2, wobei das polymere Material ausgewählt ist aus der Gruppe bestehend aus
a) einer Säule;
b) Kügelchen zum Packen einer Säule;
c) ein Filter;
d) eine Filtermembran auf entweder einer oder beiden Seiten der Membran;
e) ein Dialyse-Kappilarröhrchen;
f) eine Matrix; und
g) ein Reaktor.

4. Vorrichtung nach Anspruch 1, weiter umfassend Mittel zum Separieren von Blut in Plasma oder Ultrafiltrat.

5. Vorrichtung nach Anspruch 1, wobei das polymere Material Bindungspartner hat, die darauf immobilisiert sind, die behandelt wurden, um den lebensfähigen oder infektiösen Virus zu reduzieren.

6. Vorrichtung nach Anspruch 5, wobei das Material mittels Elektronenstrahlensterilisation behandelt wurde um Viruspartikel oder Komponenten davon zu inaktivieren oder zu vernetzen.

## Revendications

1. Dispositif stérile permettant d'éliminer le récepteur 1 soluble du facteur de nécrose tumorale (sTNFR1), le récepteur 2 soluble du facteur de nécrose tumorale (sTNFR2) et le récepteur soluble de l'interleukine 2 (sIL2R) à partir du sang ou du plasma, ledit dispositif comprenant une composition comprenant des partenaires de liaison se liant au récepteur 1 soluble du facteur de nécrose tumorale (sTNFR1), au récepteur 2 soluble du facteur de nécrose tumorale (sTNFR2) et au récepteur soluble de l'interleukine 2 (sIL2R) immobilisé sur un matériau polymère dans une colonne ou un filtre.

2. Dispositif selon la revendication 1, dans lequel le partenaire de liaison est choisi dans le groupe constitué par
a.) un anticorps polyclonal ou monoclonal se liant au sTNFR1, au sTNFR2 ou au sIL2R ;
b.) un fragment d'anticorps (anticorps à chaîne unique, recombinant ou humanisé) se liant au sTNFR1, au sTNFR2 ou au sIL2R ;
c.) une cytokine se liant au sTNFR1, au sTNFR2 ou au sIL2R ;
d.) un fragment de cytokine se liant au sTNFR1, au sTNFR2 ou au sIL2R; et
e.) un peptide synthétique se liant au sTNFR1, au sTNFR2 ou au sIL2R.

3. Dispositif selon l'une quelconque des revendications 1 ou 2, où le matériau polymère est choisi dans le groupe constitué par
a.) une colonne ;
b.) des billes pour le remplissage d'une colonne ;
c.) un filtre ;
d.) une membrane de filtre, sur l'un ou l'autre ou les deux côtés de la membrane ;
e.) un tuyau capillaire de dialyse ;
f.) une matrice ; et
g.) un réacteur.

4. Dispositif selon la revendication 1, comprenant un autre moyen pour séparer le sang en plasma ou ultrafiltrat.

5. Dispositif selon la revendication 1, où le matériau polymère ayant des partenaires de liaison immobilisés dessus a été traité pour réduire les virus viables ou infectieux.

6. Dispositif selon la revendication 5, où le matériau a été traité par une stérilisation E-Beam pour inactiver ou réticuler les particules virales ou des composants de celles-ci.
